# EUROPÄISCHE PATENTANMELDUNG

(11) **EP 4 431 824 A1**
(43) Veröffentlichungstag der Anmeldung: **18.09.2024**
(21) Anmeldenummer: 24163103.5
(22) Anmeldetag: 13.03.2024
(51) Int. Cl.: F24F 8/192, A61L 9/22, F24F 7/06, F24F 13/06, F24F 8/30, F24F 3/16

(54) **IONISIERUNGSEINRICHTUNG UND VERFAHREN ZUM REINIGEN VON RAUMLUFT**

(30) Priorität: 13.03.2023 DE 102023106128; 12.03.2024 DE 102024107046
(71) Anmelder: Schako KG, 78600 Kolbingen (DE)
(72) Erfinder: Müller, Rainer, 78600 Kolbingen (DE)
(74) Vertreter: Patentanwälte und Rechtsanwalt Weiß, Arat & Partner mbB

(57) **Zusammenfassung**

Die Erfindung betrifft ein Verfahren zum Reinigen von Raumluft von organischen und/oder anorganischen Partikeln (2), insbesondere von gesundheitsgefährdenden Keimen, die in einen Raum (1) eingetragen werden, wobei dem Raum (1) über einen Zuluftdurchlass (3) Luft zugeführt wird, soll in den Zuluftdurchlass (3) eine Ionisierungs-Einrichtung (V1) integriert werden.

## Beschreibung

### Technisches Gebiet

Die Erfindung betrifft ein Verfahren zum Reinigen von Raumluft nach dem Oberbegriff des Anspruchs 1 und eine Ionisierungs-Einrichtung nach dem Oberbegriff des Anspruchs 5.

### Stand der Technik

Insbesondere infolge der COVID-Pandemie wird der Ruf nach Lufthygiene immer größer. Dies gilt vor allem für geschlossene Räume, in denen sich eine Vielzahl von Personen aufhalten, da dort das Infektionsrisiko besonders hoch ist. In geschlossenen Räumen besteht regelmäßig die Gefahr, dass eine Übertragung von Infektionen durch im Raum verteilte Aerosole stattfindet. Die Keime werden mit der Atemluft ausgestoßen und schweben im Raum, wobei aufsteigende Luft aufgrund verschiedener Wärmequellen durch Konvektion auch nach oben steigt und die Aerosole dadurch weiter im Raum verteilt werden können. Eine Belüftung über geöffnete Fenster hat nur einen überschaubaren Effekt und wird bei niedrigen Außentemperaturen regelmäßig vernachlässigt.

Dementsprechend hat auch die Anzahl von Schutzrechten, die sich auf die Lufthygiene beziehen, schlagartig zugenommen. So zeigt beispielsweise die DE 20 2021 104 983 U1 eine Luftdesinfektionsvorrichtung. Hier weist ein Gehäuse einen Zuluftdurchlass, einen Abluftdurchlass und Luftdesinfektionsmittel auf, wobei die Luftdesinfektionsmittel zur Desinfektion, der durch das Gehäuse von Zuluftdurchlass zu Abluftdurchlass hindurchströmenden Luft eingerichtet sind, um desinfizierte Luft zu erhalten, wobei die Luftdesinfektionsmittel eine UV-Lichtquelle aufweisen, die UV-C-Licht ausstrahlt. Diese Luftdesinfektionsvorrichtung bildet eine eigenständige Einheit, die an das Verbundnetz angeschlossen werden kann.

Gemäß der EP 3 991 758 A2 wird eine Reinigung der Raumluft innerhalb eines geschlossenen Raums dadurch ermöglicht, dass ein senkrechtes Luftleitelement eingesetzt wird, durch welches die Luft hindurchgeleitet wird. Die Luft wird in einem oberen Raumbereich angesaugt und am Boden wieder ausgeblasen, wobei innerhalb des Luftleitelements eine Bestrahlung mit UV-C-Strahlung erfolgt, welche Viren zerstört und die Luft von diesen befreit.

Auch die DE 20 2022 001 585 U1 beschreibt eine Einrichtung zum Behandeln von Luft mit mindestens einem Gehäuse, in dem wenigstens ein Lüfter und mindestens eine Funktionseinheit untergebracht sind. Ein Zuluftdurchlass ist mit einem Strömungsraum strömungsverbunden, der in Strömungsrichtung der Luft hinter der Funktionseinheit liegt. Ferner ist ein Abluftdurchlass vorgesehen, in dem wenigstens eine Zusatzeinheit zum Entkeimen und/oder Desinfizieren untergebracht ist.

Vor allem ist es auch schwierig, Luftverunreinigungen, die nur von einem Objekt ausgehen, welches dem Raum hinzugefügt wird, beispielsweise von einer mit Keimen infizierten Person, welche einen Raum betritt, zu bekämpfen. Hier ist es absolut unzureichend, wenn eine Einrichtung zum Vernichten von Keimen lediglich in einem Abluftdurchlass integriert ist.

### Aufgabe der Erfindung

Die Aufgabe der vorliegenden Erfindung ist es, ein Verfahren der o.g. Art zu entwickeln, mit dem effizienter eine Luftreinigung stattfinden kann, vor allem bei gleichzeitiger Zuführung von Luft in einen Raum.

### Lösung der Aufgabe

Zur Lösung der Aufgabe führen die Merkmale nach Anspruch 1 und 5.

Zu diesem Zweck soll die Luft in dem Zuluftdurchlass so aktiviert werden, dass sie insbesondere die organischen Partikel, wie Keime, Viren, Bakterien usw. deaktiviert. D. h., dass diese aktivierte Luft die Partikel im Raum direkt bekämpft, bevor die Partikel ein gefährdetes Objekt, insbesondere eine gefährdete Person erreichen können.

Für die Aktivierung der Luft sind verschiedene Möglichkeiten bekannt, die auch für die vorliegende Erfindung anwendbar sind. Aus diesem Grunde soll die Erfindung nicht auf eine Möglichkeit beschränkt werden. Vorteilhaft für diese Erfindung ist es die Aktivierung der Luft durch eine Ionisierungsvorrichtung durchzuführen. Auf jeden Fall können beispielsweise Keime auf mechanische und/oder physikalische und/oder chemische Weise beseitigt werden. In entsprechender Weise kann auch die Luft aktiviert werden.

Die vorliegende Erfindung soll vor allem auch dazu geeignet sein, bereits vorhandene Zuluftdurchlässe nachzurüsten. Hierzu ist vorgesehen, dass die entsprechende Ionisierungs-Einrichtung entfernbar dem Zuluftdurchlass zugeordnet wird.

Die Zuluft wird im Zuluftdurchlass durch ein erfindungsgemäßes Verfahren aktiviert, um die sich im Raum befindliche, verunreinigte Luft zu reinigen. Die Raumabluft wird durch einen Abluftdurchlass über die Lüftungsanlage abgeführt.

Ein erfindungsgemässes Verfahren ist zum Reinigen von Raumluft von organischen und/oder anorganischen Partikeln vorgesehen. Dabei sollen insbesondere gesundheitsgefährdenden Keimen, die in einen Raum eingetragen werden, gereinigt werden, wobei dem Raum über einen Zuluftdurchlass Luft zugeführt wird, und in den Zuluftdurchlass eine Ionisierungs-Einrichtung integriert wird. Integriert bedeutet in diesem Zusammenhang, dass entweder in einen vorhandenen Zuluftdurchlass mittels eines Systems die Ionisierungs-Einrichtung durch entsprechende Verbindungsstellen eingesetzt oder zwischen den Zuluftdurchlass und der Zuführung der weiteren Luft zwischengeschalten wird.

Dazu kann zusätzlich vorgesehen sein, dass die aus dem Raum austretende Luft über einen Abluftdurchlass abgeführt wird. Der Abluftdurchlass kann gelichzeitig eine weiterer Zuluftdurchlass für einen weiteren Raum darstellen, sodass auch in dem Abluftdurchlass eine weitere Ionisierungs-Einrichtung vorgesehen sein kann.

Weiter kann durch die oben beschriebene Aktivierung organischer Partikel im Raum, insbesondere gesundheitsgefährdende Keime, unschädlich gemacht werden.

Zusätzlich kann durch die Aktivierung der Zuluft durch die Ionisierungs-Einrichtung die Partikel mechanisch und/oder physikalisch und/oder chemisch aus der Raumluft deaktiviert bzw. entfernt werden.

Die erfindungsgemässe Ionisierungs-Einrichtung dient ebenfalls zum Reinigen von Raumluft von organischen und/oder anorganischen Partikeln, insbesondere von gesundheitsgefährdenden Keimen, die in einen Raum eingetragen werden, wobei dem Raum über einen Zuluftdurchlass Luft zuführbar ist, wobei erfindungsgemäss in den Zuluftdurchlass eine Ionisierungs-Einrichtung integriert ist.

Zusätzlich kann eine lösbare Verbindung der Ionisierungs-Einrichtung mit dem Zuluftdurchlass vorgesehen sein. Dies ist beispielsweise durch Standardanschlüsse, Schnellspannsysteme oder mittels Verschweissen oder Fügen möglich. Dabei ist es unbeachtlich, ob Werkzeug für die Verbindung benötigt wird oder eine lösbare Verbindung ohne Werkzeug allein durch manuelle Betätigung erfolgen kann.

Weiter kann die Ionisierungs-Einrichtung mindestens eine, bevorzugt jedoch zwei oder mehrere Elektroden mit positiver und negativer Spannung zur Beseitigung der Partikel in dem Raum vorsehen.

Dazu sollte der Abstand der Elektroden ca. 15-30 mm betragen. Auf diese Weise ist eine besonders gute Reinigung der Luft, Zuluft oder Abluft möglich.

In gleicher Weise vorteilhaft ist es, wenn die Elektroden orthogonal und/oder parallel zum Luftstrom angeordnet sind.

Zusätzlich kann ein Halter für die Elektroden aus einem aerodynamischen Profil bestehen, durch das insbesondere bei großen Rohrdurchmessern eine laminare Luftströmung in mindestens zwei, bevorzugt in drei oder vier Teile aufgeteilt wird. Durch die Aufteilung wird dann wiederum ein besseres Reinigungsergebnis der Luft, Zuluft oder Abluft erreicht.

Eine ideale Strömungsgeschwindigkeit liegt bei ca. 2 bis 3 m/s im Rohr. Auf diese Weise ist ein ausreichender Aufenthalt der Luft, Zuluft oder Abluft gegeben um eine fast vollständige Reinigung zu erreichen.

Jeder Teilluftstrom weist mindestens eine, bevorzugt zwei Elektroden auf und ist/sind im Bereich der Elektroden laminar ausgebildet, um eine Rekombination der Ionen zu verhindern.

Die Ionisierungs-Einrichtung kann zusätzlich lösbar in dem Zuluftdurchlass integriert sein. Integriert kann aufgenommen, zwischengeschalten oder modular in Form eines Nachrüstsatzes nachträglich einbaubar sein.

Insbesondere soll auch die Verwendung einer Ionisierungs-Einrichtung zum Einbau in einer Belüftungsanlage für ein Gebäude geschützt sein. Diese Art von Ionisierungs-Einrichtungen sind schon in vielfältiger Form bekannt. Das Integrieren in der Belüftungsanlage eines Gebäudes ist hingegen noch nicht bekannt. Als Gebäude kommen alle Gebäude in Betracht, in welchem sich Personen vorübergehend oder dauerhaft aufhalten. Das heisst, dass beispielsweise nicht nur die zum Wohnen dienenden Gebäude in Betracht kommen, sondern auch Arbeitsplätze wie Büros, Fertigungshallen oder Bahnhöfe oder Flughäfen.

Vor allem das ursprüngliche Vorsehen oder der nachträgliche Einbau als Nachrüstsatz in einem Zuluftdurchlass oder Abluftdurchlass soll Teil der Verwendung sein.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in:
Figur 1 eine schematische Darstellung einer Raumsituation ohne Frischlufteinlasse;
Figur 2 die schematische Darstellung der Raumsituation mit zwei Personen beim Einlass von Frischluft;
Figur 3 eine schematische Darstellung der Raumsituation mit zwei Personen und einer mit einem Zuluftdurchlass verbundenen lonisierungs-Ionisierungs-Einrichtung und einem von der Zuluft getrennten Abluftdurchlass.

### Ausführungsbeispiel

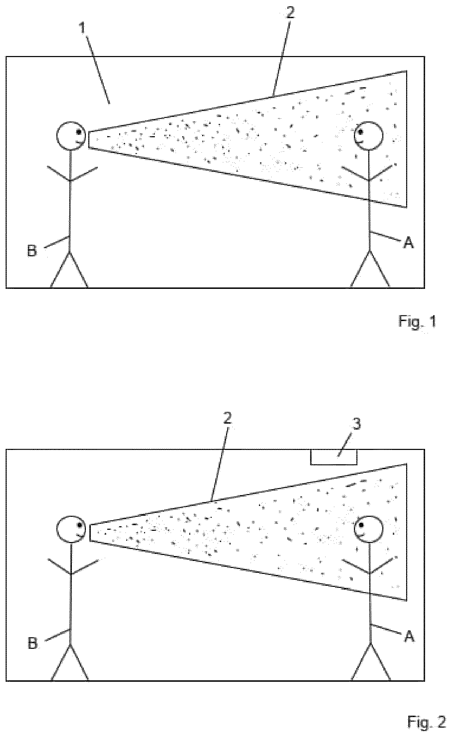

Gemäß Figur 1 befindet sich in einem Raum 1 eine gesunde Person A. Der Raum 1 wird von einer infizierten Person B betreten. Die von dieser infizierten Person B ausgehenden Keime 2 verbreiten sich im gesamten Raum 1, sodass die große Gefahr besteht, dass auch die gesunde Person A infiziert wird.

Es ist nun davon auszugehen, dass der Raum 1 gemäß Figur 2 über einen Zuluftdurchlass 3 mit Frischluft versorgt wird. Diese Frischluft vermischt sich mit der im Raum 1 vorhandenen Luft, d. h. auch mit bereits verseuchter Luft. Die Gefahr, dass sich die gesunde Person A infiziert, ist nach wie vor sehr hoch. Daran ändert sich auch nichts, wenn nur dem Zuluftdurchlass 3 eine Ionisierungs-Einrichtung zum Abtöten der Keime zugeordnet wird, da dies nichts an dem Verbleiben der verseuchten belasteten Luft im Raum 1 ändert.

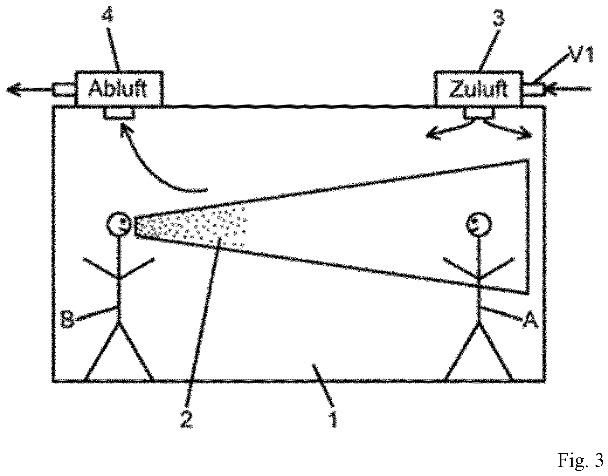

Gemäß der vorliegenden Erfindung gemäss Figur 3 soll deshalb die Luft in dem Zuluftdurchlass 3, bevorzugt in einem Anschlusskasten, durch eine Ionisierungs-Einrichtung V1 aktiviert werden. Diese Aktivierung bewirkt eine physikalische und/oder chemische Veränderung der Luft, die dazu führt, dass Keime, insbesondere organische, schädliche Keime, abgetötet werden, bevor sie eine gesunde Person A erreichen. Die aktivierte Zuluft dringt in alle Bereiche eines Raums, insbesondere in kleine Ritzen und Spalten und ermöglicht dadurch eine wirksame Reduktion von Keimen, Viren usw. in nahezu allen Bereichen. Dies ist aber nur beispielsweise.

Die Ionisierungs-Einrichtung V1 besteht aus mindestens einem System zur Ionisierung von Luft bestehend aus mindestens einer Elektrode im vorbeigeführten Luftstrom. Bevorzugt werden jedoch zwei Elektroden mit positiver und negativer Spannung verwendet. Der Abstand der Elektroden beträgt dabei 15 bis 30 mm. Die Elektroden können sowohl orthogonal als auch parallel zum Luftstrom angeordnet sein.

Als Halter oder Montagevorrichtung für die Elektroden wird in optimaler Weise ein aerodynamisch optimiertes Profil verwendet. Bei großen Rohrleitungsdurchmessern bzw. Querschnitten wird die Querschnittsfläche durch die Vorrichtung in mindestens zwei Segmente aufgeteilt. Vorzugsweise erfolgt eine Aufteilung in drei bis vier Segmente. Jeder durch die Segmentierung gebildete Teilluftstrom erhält mindestens eine Elektrode, bevorzugt zwei Elektroden.

Jeder Teilluftstrom ist zumindest im Bereich der Elektroden laminar. Dies wird unter anderem erreicht durch die Verwendung der o.g. aerodynamischen Profile der Elektrodenhalterungen. Durch die Aufteilung und die laminare Strömung kann zum einen die Anzahl der erzeugten Ladungsträger erhöht und deren Rekombination verringert werden.

Die Ionisierungs-Einrichtung V1 kann im Ganzen so ausgeführt werden, dass sie als Rohreinschub einfach auch in bestehende Rohrleitungen, insbesondere Eintrittsstutzen von z.B. Luftauslässen, eingebracht werden kann.

Die Strömungsgeschwindigkeit liegt vorteilhaft im Bereich von 2 bis 5 m/s.

Durch dieses erfindungsgemäße Verfahren wird mittels der aktivierten Frischluft aus dem Zuluftdurchlass 3 die von der infizierten Person B infizierte Luft insbesondere von organischen Partikeln gereinigt, sodass diese Luft so wenig wie möglich mit der gesunden Person A in Kontakt kommen. Sie wird dann durch einen Abluftdurchlass wieder weggeführt.

### Bezugszeichenliste

| | |
|---|---|
| 1 | Raum |
| 2 | Keime |
| 3 | Zuluftdurchlass |
| 4 | Abluftdurchlass |
| | |
| A | Gesunde Person |
| B | Infizierte Person |
| | |
| V1 | Ionisierungs-Einrichtung |

## Patentansprüche

1. Verfahren zum Reinigen von Raumluft von organischen und/oder anorganischen Partikeln (2), insbesondere von gesundheitsgefährdenden Keimen, die in einen Raum (1) eingetragen werden, wobei dem Raum (1) über einen Zuluftdurchlass (3) Luft zugeführt wird,
**dadurch gekennzeichnet,**
**dass** in den Zuluftdurchlass (3) eine Ionisierungs-Einrichtung (V1) integriert wird.

2. Verfahren nach Anspruch 1, **dadurch gekennzeichnet, dass** die aus dem Raum (1) austretende Luft über einen Abluftdurchlass (4) abgeführt wird.

3. Verfahren nach Anspruch 1 oder 2, **dadurch gekennzeichnet, dass** durch die Aktivierung organische Partikel (2) im Raum (1), wie insbesondere gesundheitsgefährdende Keime, unschädlich gemacht werden.

4. Verfahren nach wenigstens einem der vorhergehenden Ansprüche, **dadurch gekennzeichnet, dass** durch Aktivierung der Zuluft durch die Ionisierungs-Einrichtung (V1) die Partikel (2) mechanisch und/oder physikalisch und/oder chemisch aus der Raumluft deaktiviert bzw. entfernt werden.

5. Ionisierungs-Einrichtung (V1) zum Reinigen von Raumluft von organischen und/oder anorganischen Partikeln (2), insbesondere von gesundheitsgefährdenden Keimen, die in einen Raum (1) eingetragen werden, wobei dem Raum (1) über einen Zuluftdurchlass (3) Luft zuführbar ist, **dadurch gekennzeichnet, dass** in den Zuluftdurchlass (3) eine Ionisierungs-Einrichtung (V1) integriert ist.

6. Ionisierungs-Einrichtung (V1) nach Anspruch 5, **dadurch gekennzeichnet, dass** eine lösbare Verbindung der Ionisierungs-Einrichtung (V1) mit dem Zuluftdurchlass (3) vorgesehen ist

7. Ionisierungs-Einrichtung (V1) nach Anspruch 5 oder 6, **dadurch gekennzeichnet, dass** mindestens eine, bevorzugt jedoch zwei oder mehreren Elektroden mit positiver und negativer Spannung zur Beseitigung der Partikel (2) in dem Raum (1) vorgesehen sind.

8. Ionisierungs-Einrichtung (V1) nach Anspruch 7, **dadurch gekennzeichnet, dass** der Abstand der Elektroden ca. 15-30 mm beträgt.

9. Ionisierungs-Einrichtung (V1) nach Anspruch 7 oder 8, **dadurch gekennzeichnet, dass** die Elektroden orthogonal und/oder parallel zum Luftstrom angeordnet sind.

10. Ionisierungs-Einrichtung (V1) nach wenigstens einem der Ansprüche 7 bis 9, **dadurch gekennzeichnet, dass** ein Halter für die Elektroden aus einem aerodynamischen Profil besteht, durch das insbesondere bei großen Rohrdurchmessern eine laminare Luftströmung in mindestens zwei, bevorzugt in drei oder vier Teile aufgeteilt wird.

11. Ionisierungs-Einrichtung (V1) nach Anspruch 10, **dadurch gekennzeichnet, dass** eine ideale Strömungsgeschwindigkeit bei ca. 2-3 m/s im Rohr liegt.

12. Ionisierungs-Einrichtung (V1) nach Anspruch 10 oder 11, **dadurch gekennzeichnet, dass** jeder Teilluftstrom mindestens eine, bevorzugt zwei Elektroden erhält und im Bereich der Elektroden laminar ausgebildet ist, um eine Rekombination der Ionen zu verhindern.

13. Ionisierungs-Einrichtung (V1) nach wenigstens einem der Ansprüche 5 bis 12, **dadurch gekennzeichnet, dass** sie lösbar in dem Zuluftdurchlass (3) integriert ist.

14. Verwendung einer Ionisierungs-Einrichtung (V1) nach den Ansprüchen 5 bis 13, **gekennzeichnet durch** den Einbau in einer Belüftungsanlage für ein Gebäude.

15. Verwendung nach Anspruch 14, **gekennzeichnet durch** den ursprünglichen oder nachträglichen Einbau in einem Zuluftdurchlass oder Abluftdurchlass.
